# EUROPÄISCHE PATENTSCHRIFT

(11) **EP 0 397 599 B1**
(45) Veröffentlichungstag und Bekanntmachung des Hinweises auf die Patenterteilung: **09.03.1994**
(21) Anmeldenummer: 90810335.1
(22) Anmeldetag: 02.05.1990
(51) Int. Cl.: G03B 42/04, A61B 6/14

(54) **Röntgenfilmhalter-Satz für die Aufnahme von Röntgenbildern eines ganzen Zahnes**
X-ray film support assembly for the taking of X-ray photographs of a complete tooth
Ensemble de support pour roentgen-film pour l'exposition d'épreuves radiographiques d'une dent entière

(30) Priorität: 10.05.1989 CH 1757/89
(43) Veröffentlichungstag der Anmeldung: 14.11.1990
(73) Patentinhaber: Klauser, Rolf M., Dr., CH-6010 Kriens (CH)
(72) Erfinder: Klauser, Rolf M., Dr., CH-6010 Kriens (CH)
(74) Vertreter: Seehof, Michel

(56) Entgegenhaltungen:
- EP-A- 0 279 955
- EP-A- 0 307 617
- US-A- 3 304 422
- US-A- 4 057 732
- US-A- 4 251 732
- US-E- 25 773

## Beschreibung

Die vorliegende Erfindung bezieht sich auf einen Röntgenfilmhalter-Satz gemäss Oberbegriff von patentanspruch 1. Aus der EP-A-0 279 955 ist ein Filmhalter für Messröntgenbilder anlässlich von Wurzelbehandlung bekannt, wobei die dort offenbarte Erfindung darauf gerichtet ist, die bei der Wurzelbehandlung eingesteckten Instrumente bei der Röntgenaufnahme zu berücksichtigen. Der Halter ist einerseits nur für Aufnahmen der Seitenzähne und andererseits nur für Aufnahmen mittels einem Langtubus offenbart und ausgerüstet. Allein für die Aufnahme der Seitenzähne sind zwei Filmhalter notwendig, die vom Indikatorstab abgenommen und umgesteckt werden müssen.

Aus der US-A-3 003 062 ist ferner ein Filmhalter mit einer Zentriervorrichtung bekannt, mit der sämtliche Zähne geröntgt werden können. Dazu weist der Halter einen in seiner Ebene drehbaren Schenkel auf, um entweder die rechte oder die linke Mundseite resp. die unteren oder oberen Zähne aufnehmen zu können. Als Aufbiss wird ein steckbarer Teil zusammen mit einer Watterolle verwendet. Ausserdem muss dieser Halter beim Wechsel von den oberen zu den unteren Zähnen auseinander genommen und umgesteckt werden.

Aus der EP-A-078 425 ist ein weiterer Halter für die Aufnahme aller Zähne bekannt, wobei dieser Halter modular aus verschiedensten Teilen aufgebaut ist, die je nach Bedarf umgesteckt werden müssen.

Es ist demgegenüber Aufgabe der vorliegenden Erfindung einen Röntgenfilmhalter-Satz für sämtliche intra-oralen Röntgenfilmformate anzugeben, der es ermöglicht, alle Seitenzähne, resp. Frontzähne ohne Teile ab- oder zusamenbauen zu müssen, aufzunehmen und der aus wenigen Teilen kostengünstig hergestellt werden kann. Ein solcher Röntgenfilmhalter-Satz ist in Patentanspruch 1 definiert.

Mit einer Weiterausbildung der Erfindung gemäss Patentanspruch 2 ist es ausserdem möglich, für alle vorkommenden Röntgentuben wie Kurz- und Langtuben, zylindrische, konische und eckige, spitze und vorne flache Tuben für eine gute Führung und Zentrierung des Röntgenzentralstrahles Gewähr zu bieten.

Die Erfindung wird nun anhand einer Zeichnung von Ausführungsbeispielen näher erläutert.
- Fig. 1: zeigt in Seitenansicht einen erfindungsgemässen Röntgenfilmhalter für die Aufnahme von Seitenzähnen im Munde eines Patienten,
- Fig. 2: zeigt den Halter von Fig. 1 in perspektivischer Sicht,
- Fig. 3: zeigt den Halter von Fig. 2 von rechts,
- Fig. 4: zeigt einen Ausschnitt des Halters von Fig. 3,
- Fig. 5: zeigt einen Teil des Halters gemäss Fig. 3 von oben,
- Fig. 6: zeigt eine Seitenansicht des erfindungsgemässen Röntgenfilmhalters für die Frontzähne im Munde eines Patienten,
- Fig. 7: zeigt den Röntgenfilmhalter gemäss Fig. 6 in perspektivischer Sicht und
- Fig. 8: zeigt eine Ausführungsvariante der vorhergehenden Röntgenfilmhalter.

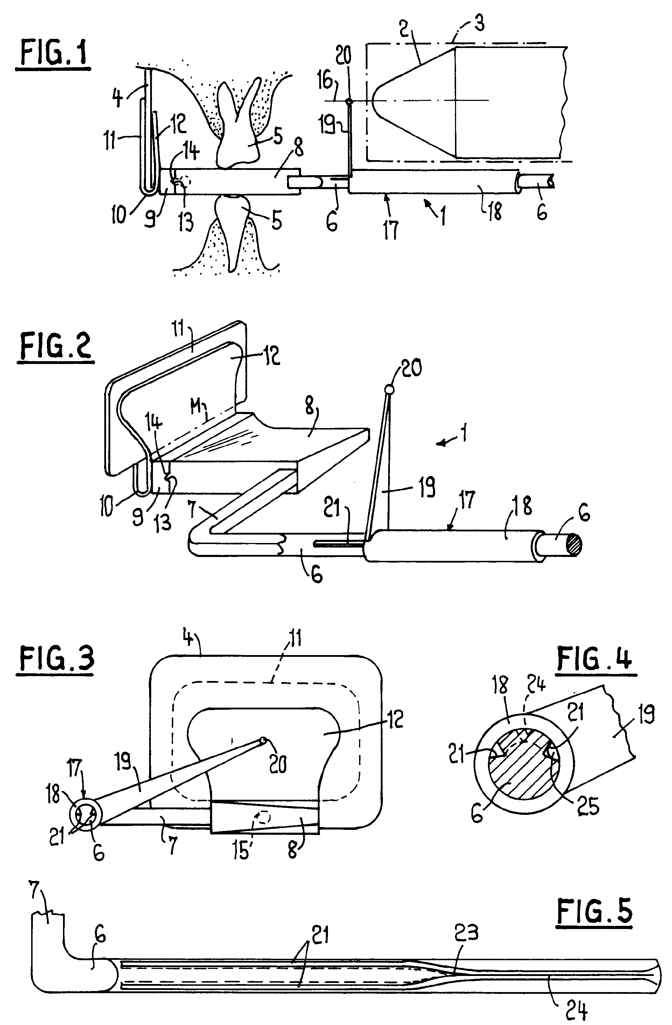

In Fig. 1 erkennt man den ersten Röntgenfilmhalter 1 für die Aufnahme von Seitenzähnen, einen konischen Röntgentubus 2 sowie einen strichpunktiert eingezeichneten zylindrischen Röntgentubus 3. Der Röntgenfilmhalter 1 trägt intra-oral den Röntgenfilm 4, der sich hinter den zwei Seitenzähnen 5 befindet. Die wesentlichen Teile des Röntgenfilmhalters 1 sind der Indikatorstab 6 mit seiner Abkröpfung 7 und wieder senkrecht zur Abkröpfung 7 der Aufbissblock 8, dem sich über ein drehbar am Aufbissblock befestigten Zwischenstück 9 die federnde Filmklemme 10 anschliesst. Die federnde Filmklemme 10 ist U-förmig ausgebildet und weist eine Rückenplatte 11 und einen etwas kleineren Klemmschenkel 12 auf, der jedoch so gross ist, dass der Röntgenfilm vor dem Verbiegen geschützt ist. Auf der Rückseite der Rückenplatte ist eine Markierung M angebracht, um alle drei gebräuchlichen Filmformate exakt zu zentrieren. Die Markierung ist derart, dass die Filmformate Nr. 0 und Nr. 1 bis zur Markierung eingeschoben werden, während das Filmformat Nr. 2 ganz eingeschoben wird. Aus Fig. 2 geht hervor, dass der Aufbissblock nicht als parallele Platte ausgebildet ist, sondern vorne, d.h. bei der Abkröpfung 7 dicker ist als hinten sowie oben und unten leicht konkav gewölbt und auf beiden Seiten etwas angerauht ist. Zwecks Justierung der Filmklemme 10 bezüglich dem Aufbissblock weist dieser eine Feder 13 und das Zwischenstück 9 eine Nut 14 auf, wobei diese Rastmittel selbstverständlich auch umgekehrt ausgebildet sein können. Die in Fig. 3 schematisch angedeutete Drehachse 15 kann in geeigneter Weise im Zwischenstück und/oder im Aufbissblock befestigt sein, beispielsweise als eingeklemmte Verdickung.

Im Prinzip wäre es möglich, mit einem solchen Röntgenfilmhalter Aufnahmen der Seitenzähne zu machen, falls man einen bestimmten, nämlich zylindrischen, Tubustyp verwendet. Werden jedoch andere Tubustypen verwendet ist es notwendig, den Röntgenzentralstrahl 16 (siehe Fig. 1) genau auf den Filmmittelpunkt auszurichten, wobei für alle drei Filmformate der gleiche Mittelpunkt gilt.

Dazu wird ein Index 17 benötigt, der ein Rohr 18 mit einem senkrecht dazu stehenden Zeiger 19 mit Indexspitze 20 enthält. Dabei ist die Länge des Zeigers 19 derart, dass dessen Spitze 20 genau dem Mittelpunkt aller drei Filmformate in der genau eingezeichneten Stellung gemäss dem Mittelpunkt der Rückenplatte des Filmhalters entspricht.

Da die Filmklemme in zwei um 180° gedrehte Stellungen, d.h. nach oben oder nach unten einrastbar ist, muss auch der Index, respektive dessen Spitze 20 auf den Mittelpunkt der Filme in beiden Stellungen weisen können. Zu diesem Zwecke weist der Indikatorstab 6 zwei Führungsrillen 21 und 22 auf, die sich im hinteren Teil des Indikatorstabes bei der Abzweigung 23 zu einer einzigen Rille vereinen, wobei diese einzige Rille 24 zwischen den beiden anderen Rillen angeordnet ist, wie aus den Fig. 4 und 5 hervorgeht. Aus Fig. 4 geht ferner hervor, dass das Rohr 17 des Indexes einen Nocken 25 aufweist, der in die Rillen passt. Es ist selbstverständlich, dass auch die Umkehrung gilt, d.h. dass der Indikatorstab einen Nocken aufweist und das Rohr des Indexes zwei entsprechende Rillen. Zu jeder Stellung der Filmklemme oben oder unten kann der Index entsprechend eingestellt werden, wobei das Rohr nach rückwärts gezogen wird, bis der Nocken in die vereinte Rille 24 greift, wonach das Rohr in die entsprechende Rille geschoben werden kann. In beiden Stellungen weist die Spitze 20 des Indexes genau auf die Mitte der Rücken- platte und somit genau auf die Mitten aller drei Filmformate.

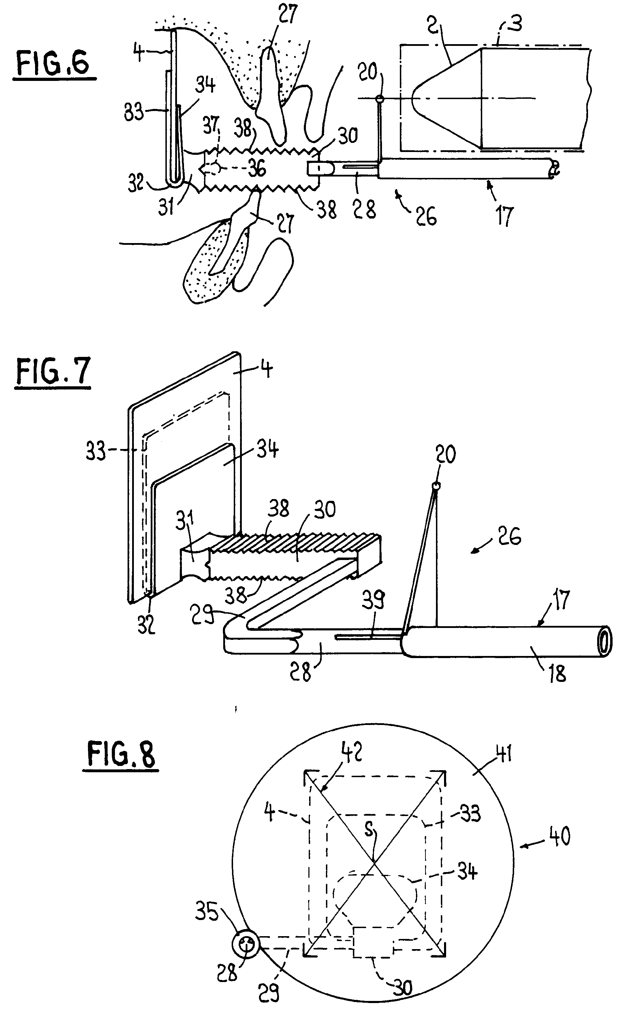

In den Fig. 6 und 7 ist der zweite Röntgenfilmhalter 26 des Satzes dargestellt, der der Aufnahme von Frontzähnen dient. Man erkennt wieder die beiden Röntgentuben 2 oder 3 sowie die Frontzähne 27. Auch dieser Röntgenfilmhalter besteht im wesentlichen aus dem Indikator 28 mit dem abgekröpften Teil 29, dem daran anschliessenden Aufbissblock 30 sowie das daran drehbar befestigte Zwischenstück 31, an dem sich die U-förmige Filmklemme 32 anschliesst, die eine Rückenplatte 33 sowie einen Klemmschenkel 34 aufweist. Ausserdem erkennt man einen eingeschobenen Röntgenfilm 4. Die Rückenplatte 33 entspricht in ihren Abmessungen genau dem Filmformat Nr. 0. Alle drei Filmformate 0, 1, 2 werden ganz eingeschoben, wobei die Formate 1 und 2 seitlich und oben etwas über die Rückenplatte herausragen. Die Klemmschenkel sowohl dieser Filmklemme als auch der für den Seitenhalter sind möglichst dünn auszuführen und die Oberkante ist jeweils nach innen abgeschrägt. Selbstverständlich sind alle Ecken gerundet, um keine Verletzunqen hervorzurufen. Die drehbare Befestigung des Zwischenstücks 31 am Aufbissblock 30 ist die gleiche wie beim vorhergehend beschriebenen Seitenhalter mit der gleichen Feder 13 und Nut 14, und der Befestigung durch einen verdickten Zapfen 36 in einer entsprechenden Bohrung 37 im Aufbissblock. Der Aufbissblock 30 des Fronthalters ist etwa gleichmässig dick und weist an seinen beiden Oberflächen eine Riffelung 38 auf. Der Index 17 für diesen Fronthalter ist derselbe wie für den Seitenhalter, während die Führungsrillen, wovon nur eine, 39, sichtbar ist, einen anderen Abstand voneinander aufweisen als beim Seitenhalter, da die Indexspitze nicht in die Mitte der Rückenplatte zeigt wie beim Seitenhalter, sondern in die Mitte des Filmformates Nr. 2, wenn dieses eingesteckt und bezüglich des Rückens genau zentriert ist. Aus den Fig. 2 und 7 geht ausserdem hervor, dass der Indikatorstab 6, bzw. 38 rund sein muss, während die Abkröpfung 7 oder 29 auch eckig sein kann.

Wie bezüglich des Seitenhalters angegeben, kann das Prinzip der Führungsrille und des Führungsnockens im beweglichen Index auch umgetauscht werden, d.h. dass am Indikatorstab eine einzige Führungsrippe sein kann, während der Index zwei Führungsrillen aufweisen muss für die beiden Stellungen. Da die Spitzen des Indexes für beide Halter nicht den gleichen Winkel umschliessen bzw. einen anderen Abstand voneinander aufweisen, da auf verschiedene Punkte gezielt wird, muss der Index, für den Fall, dass sich die Führungsrillen im Index befinden, für den Seitenhalter ein anderer sein als für den Fronthalter. Es ist dann zweckmässig, die beiden Halter entweder verschiedenfarbig auszuführen oder auf geeignete andere Weise zu identifizieren.

Der vorgehend beschriebene Index mit Zeiger und Zeigerspitze ist sehr gut geeignet für konische Tuben, für zylindrische Tuben jedoch ein bisschen weniger präzis, wenn auch ausreichend. In Fig. 8 ist eine Variante dargestellt, die für zylindrische Tuben sehr gut geeignet ist, aber auch für konische Tuben. Man erkennt in Fig. 8 den Index 40, der ein Rohr 35 aufweist und der zusammen mit dem entsprechenden Indikatorstab entweder einen Führungsnocken aufweist oder zwei Führungsrillen, und je nach Verwendung für einen Seitenhalter oder für einen Fronthalter angepasst ist. Statt eines Indexzeigers weist dieser Index eine Scheibe 41 auf, auf der ein Kreuz 42 aufgezeichnet ist. Die Scheibe ist an ihrem Umfang am Rohr 35 des Index befestigt. Der Umfang der Scheibe dient dabei als Zentrierungshilfe für zylindrische Tuben, während die Ecken des Kreuzes 42 als Zentrierungshilfe für eckige Tuben dient. Der Schnittpunkt S des Kreuzes dient als Zentrierungshilfe für konische Tuben und ersetzt die Spitze des Indexzeigers. Gestrichelt eingezeichnet erkennt man ferner den Klemmschenkel 32, die Rückenplatte 33 sowie den eingeschobenen Film 4.

Im allgemeinen werden sämtliche Teile der Röntgenfilmhalter aus Kunststoff gefertigt und zwar so, dass sie insbesondere aus hygienischen Gründen für die Einmalverwendung vorgesehen sein können, oder zum Sterilisieren bestimmt sind.

## Patentansprüche

1. Röntgenfilmhalter-Satz für die intraorale Aufnahme von Röntgenbildern von Zähnen, dadurch gekennzeichnet, dass er aus einem Seitenhalter (1) für die Röntgenaufnahme von Seitenzähnen und aus einem Fronthalter (26) für die Röntgenaufnahme von Frontzähnen besteht, wobei die Halter (1, 26) je einen geformten Aufbissblock (8, 30), eine federnde Filmklemme (10, 32) zur Halterung des Röntgenfilms (4) in einer Filmebene und einen senkrecht zur Filmebene stehenden, abgekröpften Indikatorstab (6, 28) zur Justierung des Röntgenzentralstrahles (16) senkrecht zur Filmebene aufweisen, und die Filmklemme (10, 32) auf der dem Indikatorstab gegenüberliegenden Seite des Aufbissblocks um 360° in der Filmebene drehbar befestigt und in zwei Positionen einrastbar ist, die zu der von der Abkröpfung des Indikatorstabs definierten Ebene symmetrisch liegen.

2. Röntgenfilmhalter-Satz nach Anspruch 1, dadurch gekennzeichnet, dass die Halter (1,26) einen Index (17) mit einem auf dem Indikatorstab (6,28) gleitbaren Rohr (18,35) aufweisen, an dem Anzeigemittel (19,20,40) befestigt sind, die als Bezugspunkt zur Ausrichtung des Röntgenzentralstrahles (16) auf die Mitte der Rückenplatte (11,33) der Filmklemme beim Seitenhalter (1) oder auf die Mitte eines auf der Rückenplatte einlegbaren Röntgenfilms (4) des Formats Nr. 2 beim Fronthalter (26) zu dienen, wobei der Indikatorstab (6,38) und das Rohr (18,35) des Indexes (17) zueinander komplementäre Mittel (21,22,23, 24;25) aufweisen, um die Anzeigemittel (19,20;40) in die den Einrastpositionen der Filmklemme jeweils entsprechenden Positionen zu bringen.

3. Röntgenfilmhalter-Satz nach Anspruch 2, dadurch gekennzeichnet, dass jeder der Indikatorstäbe (6,28) je zwei Führungsrillen (21,22;39) aufweist, die über eine Abzweigung (23) zu einer einzigen Rille (24) vereint sind und das Rohr (18,35) des gemeinsamen Indexes (17) einen den Führungsrillen entsprechenden Führungsnocken (25) aufweist, wobei die Führungsrillen am Indikatorstab (6) für den Seitenhalter (1) einen anderen Abstand voneinander haben als diejenigen des Fronthalters (26).

4. Röntgenfilmhalter-Satz nach Anspruch 2, dadurch gekennzeichnet, dass beide Indikatorstäbe (6,28) jeder der einen Führungsnocken aufweit und jedem Indikatorstab ein Index zugeordnet ist, dessen Rohr zwei Führungsrillen aufweist, wobei die Führungsrillen an dem einen Rohr einen anderen Abstand voneinander haben als am anderen Rohr.

5. Röntgenfilmhalter-Satz nach Anspruch 2, dadurch gekennzeichnet, dass die Anzeigemittel einen am Rohr (18) des Indexes (17) angeordneten und darauf senkrecht stehenden Zeiger (19) enthalten, dessen Länge derart ist, dass dessen Spitze (20) als Bezugspunkt zur Ausrichtung des Röntgenzentralstrahles (16) dient.

6. Röntgenfilmhalter-Satz nach Anspruch 2, dadurch gekennzeichnet, dass die Anzeigemittel eine am Rohr (35) des Indexes angeordnete Scheibe (40) mit einem eingezeichneten Kreuz (42) enthalten, wobei der Umfang der Scheibe (40) als Zentrierungshilfe für zylindrische Röntgentuben dient, die Ecken des Kreuzes als Zentrierungshilfe für rechteckige Röntgentuben und der Schnittpunkt (S) des Kreuzes als Zentrierungshilfe für konische Tuben dienen.

7. Röntgenfilmhalter-Satz nach einem der Ansprüche 1 bis 6, dadurch gekennzeichnet, dass der Aufbissblock (8) für den Seitenhalter (1) auf der Seite der Abkröpfung (7) des Indikatorstabes (6) eine grössere Dicke aufweist als auf der anderen Seite und beidseitig leicht konkav gewölbt und aufgerauht ist.

8. Röntgenfilmhalter-Satz nach einem der Ansprüche 1 bis 7, dadurch gekennzeichnet, dass der Aufbissblock (30) für den Fronthalter (26) einen etwa rechteckigen Querschnitt und auf beiden Seiten eine Riffelung (38) aufweist.

9. Röntgenfilmhalter-Satz nach einem der Ansprüche 1 bis 8, dadurch gekennzeichnet, dass die Rastmittel zwischen dem Aufbissblock (8,30) und dem an der Filmklemme (10, 32) angeordneten Zwischenstück (9,31) aus einer Feder (13) und einer entsprechenden Nut (24) bestehen.

## Claims

1. Set of X-ray film holders for intraoral X-ray exposures of teeth, characterised in that said set consists of a side holder (1) for X-ray exposures of side teeth and of a front holder (26) for X-ray exposures of front teeth, each of said holders (1, 26) comprising a shaped bite block (8, 30), a resilient film clip (10-32) for attaching the X-ray film (4) in a film plane, and a cranked indicator rod (6, 28) extending perpendicularly with respect to the film plane and serving to adjust the central X-ray beam (16) orthogonally with respect to said film plane, and in that said film clip (10, 32) is secured to said bite block in such a manner as to turn over 360° in said film plane and to snap into two positions which are symmetrical with respect to the plane defined by the cranked portion of said indicator rod.

2. Set of X-ray film holders according to claim 1, characterised in that said holders (1, 26) comprise an index (17) having a tube (18, 35) which is slidable on said indicator rod (6, 28) and to which indicator means (19, 20, 40) are attached which serve as a reference for aligning the central X-ray beam (16) with the center of the back plate (11, 33) of said film clip, in the case of said side holder (1), or with the center of an X-ray film (4) having the format no. 2 which is attachable to the back plate, in the case of said front holder (26), said indicator rod (6, 38) and said tube (18, 35) of said index (17) being provided with mutually complementary means (21, 22, 23, 24; 25) in order to bring said indicator means (19, 20; 40) into positions corresponding to the respective snap-in positions of said film clip.

3. Set of X-ray film holders according to claim 2, characterised in that each one of said indicator rods (6, 28) is provided with two guiding grooves (21, 22; 39) which are united by a junction (23) to form a single groove (24), and in that said tube (18, 35) of the common index is provided with a guiding nose (25) corresponding to said guiding grooves, said guiding grooves of said indicator rod (6) of the holder (1) for the side teeth having a different distance from each other than those of the front holder (26).

4. Set of X-ray film holders according to claim 2, characterised in that both of said indicator rods (6, 28) each comprise a guiding nose and in that to each indicator rod is associated an index whose tube has two guiding grooves, the guiding grooves of one of said tubes having a different distance from each other than those of the other tube.

5. Set of X-ray film holders according to claim 2, characterised in that said indicator means comprise a pointer (19) which is attached to said tube (18) of said index (17) in an orthogonal position and whose length is such that its point (20) serves as a reference for aligning the central X-ray beam (16).

6. Set of X-ray film holders according to claim 2, characterised in that said indicator means comprise a disk (40) attached to said tube (35) of said index and having a cross (42) drawn thereon, the circumference of said disk (40) serving as a centering aid for cylindrical X-ray tubes, the corners of said cross serving as a centering aid for rectangular X-ray tubes, and the intersection (S) of said cross serving as a centering aid for conical tubes.

7. Set of X-ray film holders according to any one of claims 1 to 6, characterised in that the bite block (8) of said side holder (1) is thicker on the side of said cranked portion (7) of said indicator rod (6) than on the other side and is slightly concavely curved and roughened on both sides.

8. Set of X-ray film holders according to any one of claims 1 to 7, characterised in that the bite block (30) of said front holder (26) has an approximately rectangular cross-section and ribbed areas (38) on both sides.

9. Set of X-ray film holders according to any one of claims 1 to 8, characterised in that said snap means between said bite block (8, 30) and the connecting piece (9, 31) attached to said film clip (10, 32) are formed of a key (13) and a corresponding groove (24).

## Revendications

1. Ensemble de supports pour films pour radiographies intraorales de dents, caractérisé en ce qu'il est composé d'un support latéral (1) pour radiographies de dents latérales et d'un support frontal (26) pour radiographies de dents frontales, lesdits supports (1, 26) comprenant chaque fois un bloc à mordre formé (8, 30), une pince à film (10, 32) élastique pour maintenir le film radiographique (4) dans un plan du film et une tige indicatrice (6, 28) perpendiculaire audit plan du film et coudée, pour ajuster le rayon X central (16) perpendiculairement audit plan du film, et en ce que la pince à film (10, 32) est attachée sur le côté du bloc à mordre opposé à la tige indicatrice de manière à tourner sur 360° dans le plan du film et encliquetable dans deux positions symétriques par rapport au plan défini par le coude de ladite tige indicatrice.

2. Ensemble de supports pour films radiographiques selon la revendication 1, caractérisé en ce que les supports (1, 26) comportent un index (17) avec un tube (18, 35) glissant sur la tige indicatrice (6, 28), auquel tube sont attachés des moyens indicateurs (19, 20, 40) servant de référence pour aligner le rayon X central (16) sur le centre de la plaque arrière (11, 33) de la pince à film, dans le cas du support latéral (1), ou sur le centre d'un film radiographique (4) du format no. 2 attachable sur la plaque arrière, dans le cas du porte-films frontal (26), la tige indicatrice (6, 38) et le tube (18, 35) de l'index (17) présentant des moyens mutuellement complémentaires (21, 22, 23, 24; 25) pour mettre les moyens indicateurs (19, 20; 40) dans des positions correspondant chaque fois aux dites positions d'arrêt de la pince à film.

3. Ensemble de supports pour films radiographiques selon la revendication 2, caractérisé en ce que chacune des tiges indicatrices (6, 28) présente deux rainures de guidage (21, 22; 39) qui sont unies par une jonction (23) pour former une seule rainure (24), et que le tube (18, 35) de l'index commun (17) présente un ergot de guidage (25) correspondant aux rainures de guidage, les rainures de guidage de la tige indicatrice (6) du support latéral (1) étant à une autre distance mutuelle que celles du support frontal (26).

4. Ensemble de supports pour films radiographiques selon la revendication 2, caractérisé en ce que les deux tiges indicatrices (6, 28) présentent chacune un ergot de guidage qu'à chaque tige indicatrice est associé un index dont le tube présente deux rainures de guidage, les rainures de guidage d'un des tubes étant à une autre distance mutuelle que celles de l'autre tube.

5. Ensemble de supports pour films radiographiques selon la revendication 2, caractérisé en ce que lesdits moyens indicateurs comportent une aiguille (19) attachée au tube (18) de l'index (17) et perpendiculaire à celui-ci, dont la longueur est telle que sa pointe (20) sert de référence pour aligner le rayon X central (16).

6. Ensemble de supports pour films radiographiques selon la revendication 2, caractérisé en ce que lesdits moyens indicateurs comportent un disque (40) attaché au tube (35) dudit index et portant une croix (42) dessinée, la circonférence du disque (40) servant d'aide de centrage pour tubes radiographiques cylindriques, les coins de la croix servant d'aide de centrage pour tubes radiographiques rectangulaires et l'intersection (S) de la croix servant d'aide de centrage pour tubes coniques.

7. Ensemble de supports pour films radiographiques selon l'une quelconque des revendications 1 à 6, caractérisé ce que le bloc à mordre (8) du support latéral (1) est plus épais sur le côté du coude (7) de la tige indicatrice (6) que sur l'autre côté, et qu'il est légèrement voûté en forme concave et rugueux des deux côtés.

8. Ensemble de supports pour films radiographiques selon l'une quelconque des revendications 1 à 7, caractérisé en ce que le bloc à mordre (30) du support frontal (26) présente une coupe transversale approximativement rectangulaire ainsi qu'une cannelure (38) des deux côtés.

9. Ensemble de supports pour films radiographiques selon l'une quelconque des revendications 1 à 8, caractérisé en ce que les moyens d'arrêt entre le bloc à mordre (8, 30) et la partie intermédiaire (9, 31) attachée à la pince à film (10, 32) sont formés d'une languette (13) et d'une rainure (24) correspondante.
